# EUROPÄISCHE PATENTSCHRIFT

(11) **EP 0 643 901 B1**
(45) Veröffentlichungstag und Bekanntmachung des Hinweises auf die Patenterteilung: **25.09.1996**
(21) Anmeldenummer: 93908819.1
(22) Anmeldetag: 27.04.1993
(51) Int. Cl.: H05G 1/44, A61B 6/14, H04N 5/32

(54) **ZAHNÄRZTLICHE RÖNTGENDIAGNOSTIKEINRICHTUNG**
DENTAL X-RAY DIAGNOSTIC DEVICE
INSTALLATION DE DIAGNOSTIC DENTAIRE PAR RAYONS X

(30) Priorität: 01.06.1992 DE 4218020
(43) Veröffentlichungstag der Anmeldung: 22.03.1995
(73) Patentinhaber: SIEMENS AKTIENGESELLSCHAFT, 80333 München (DE)
(72) Erfinder: BLASCHKA, Eriks, D-6940 Weinheim (DE); SCHULZE-GANZLIN, Ulrich, D-6143 Lorsch (DE)
(86) Internationale Anmeldenummer: DE9300368
(87) Internationale Veröffentlichungsnummer: WO9325059

(56) Entgegenhaltungen:
- EP-A- 0 415 075
- WO-A-92/22188

## Beschreibung

Die Erfindung bezieht sich auf eine zahnärztliche Röntgendiagnostikeinrichtung, enthaltend einen Röntgenstrahler, einen diametral dazu und in Strahlungsrichtung nach dem zu durchstrahlenden Objekt angeordneten CCD-Bilddetektor, eine Steuerelektronik mit einem Steuerteil zur Ansteuerung des Bilddetektors, eine Bildverarbeitungseinheit, welche die vom Bilddetektor gewonnenen Signale aufbereitet und einer Bilddarstellungseinheit zuführt und eine Bedienvorrichtung, mit der der Röntgenstrahler ein- und ausschaltbar ist.

Eine solche Röntgendiagnostikeinrichtung ist aus der EP-A-O 415 075 bekannt. Bei dieser bekannten Röntgendiagnostikeinrichtung ist, um insbesondere das Verbindungskabel zwischen Röntgenstrahler und der Steuerelektronik mit der Bildverarbeitungseinheit entbehrlich zu machen, im Empfangsbereich des Bilddetektors ein strahlungsempfindlicher Sensor angeordnet. Gemäß einer vorteilhaften Ausgestaltung dieser bekannten Einrichtung kann der Sensor auch innerhalb der Fläche des Bilddetektors angeordnet sein.

Der im Anspruch 1 angegebenen Erfindung liegt die Aufgabe zugrunde, demgegenüber eine weitere Verbesserung und Vereinfachung anzustreben.

Dadurch, daß der Bilddetektor kontinuierlich ausgelesen und damit die ankommenden Bilddaten ständig einer Schwellenwertüberprüfung unterzogen werden, die das Vorhandensein einer Strahlung sofort erkennt und entsprechend die Steuerelektronik einschaltet, kann auf den beim Stand der Technik im Bereich des Bilddetektors vorgesehenen strahlungsempfindlichen Sensor verzichtet werden. Die Steuerelektronik wird automatisch eingeschaltet, sobald der Röntgenstrahler über die Bedienvorrichtung eingeschaltet wird.

In der nicht vorveröffentlichten WO-A-9 222 188 ist eine Einrichtung beschrieben, bei der anstelle eines röntgenstrahlenempfindlichen Films ein Bilddetektor verwendet ist; bei dieser Einrichtung werden jedoch keine Bilddaten getaktet und ausgewertet; vielmehr werden dort die statischen Signale, welche dem zu und von dem CCD-Sensor fließenden Versorgungsstrom entsprechen, kontinuierlich einer Schwellwertüberprüfung unterzogen.

Ein Ausführungsbeispiel der Erfindung wird nachfolgend anhand der Zeichnung näher erläutert.

Es zeigen:

Figur 1 eine Übersichtsdarstellung der erfindungsgemäßen Röntgendiagnostikeinrichtung,

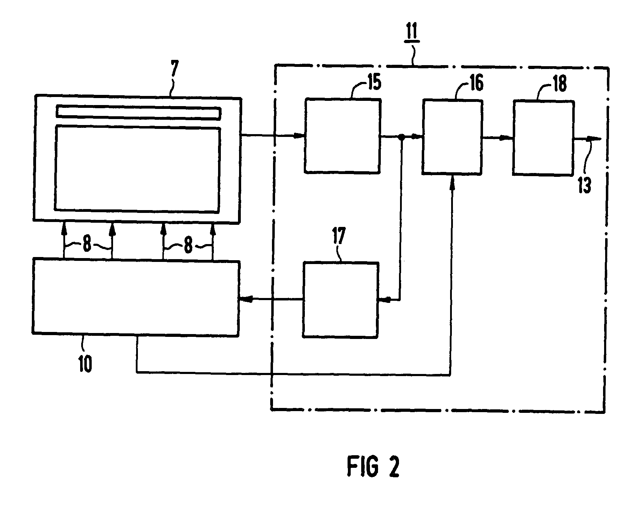

Figur 2 ein Blockschaltbild der erfindungsgemäßen Diagnostikeinrichtung.

Die Diagnostikeinrichtung enthält einen Röntgenstrahler 1, der in bekannter Weise eine nicht näher bezeichnete Röntgenröhre mit Hochspannungsgenerator enthält, und der mittels einer Halterung 2 in geeigneter Weise so gehaltert ist, daß er auf das zu durchstrahlende Objekt 3 (Zahn bzw. Kiefer eines Patienten) ausrichtbar ist. Die Halterung 2 kann im Falle einer intraoralen Diagnostikeinrichtung in bekannter Weise aus einem Boden- oder Deckenstativ, an dem der Röntgenstrahler in Höhe Neigung und Schwenkrichtung einstellbar ist, bestehen. Im Falle einer Panorama-Röntgendiagnostikeinrichtung enthält der Halter auch noch den in Strahlungsrichtung nach dem durchstrahlten Objekt angeordneten, mit 7 bezeichneten Bilddetektor.

Der Röntgenstrahler 1 enthält ferner ein mit 4 bezeichnetes Steuerteil, welches über ein Steuerkabel 5 mit einer Bedienvorrichtung 6 verbunden ist, mit deren Hilfe bestimmte Aufnahmewerte, wie Röhrenspannung, Belichtungszeit, einstellbar sind.

Dem Röntgenstrahler diametral gegenüberliegend und in Strahlungsrichtung nach dem zu durchstrahlenden Objekt 3 ist ein Bilddetektor 7 angeordnet, der im Falle einer Intraoral-Röntgendiagnostikeinrichtung im Patientenmund plazierbar ist. Der Bilddetektor besteht hier aus einem CCD-Detektor, welcher über ein Steuerkabel 8 mit einem Taktgenerator 10 verbunden ist, der Teil einer allgemein mit 9 bezeichneten Steuerelektronik ist. Diese Steuerelektronik enthält ferner noch eine Bildverarbeitungseinheit 11, die über eine Steuerleitung 12 mit dem CCD-Detektor verbunden ist und die vom Detektor gewonnenen Signale entsprechend aufbereitet, abspeichert, verstärkt und schließlich über eine Leitung 13 einer Bilddarstellungseinheit 14, z.B. einem Monitor 14, zuführt.

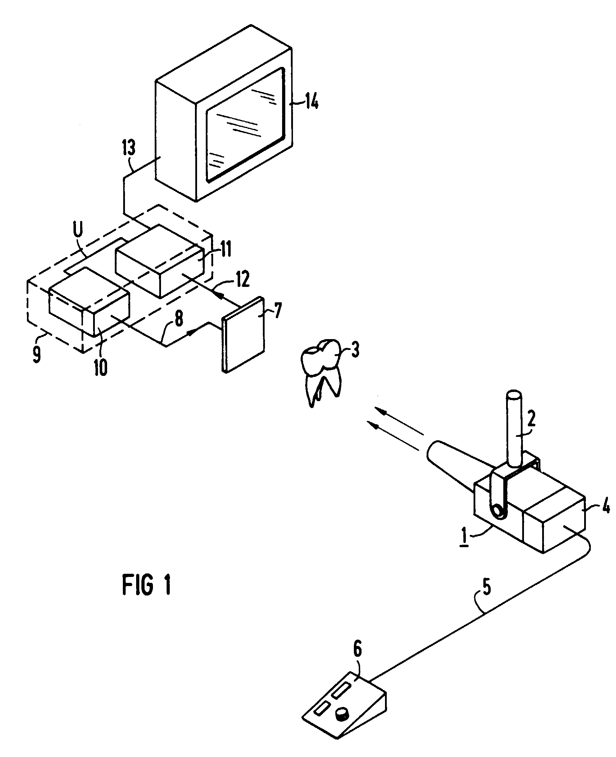

Erfindungsgemäß wird der CCD-Detektor 7 über den Taktgenerator 10 kontinuierlich ausgelesen, wobei die ankommenden Bilddaten in einem Schwellenwertschalter 17 ständig einer Schwellenwertüberprüfung unterzogen werden. Im sogenannten 'Stand-by'-Zustand, in dem der Röntgenstrahler 1 ausgeschaltet ist, wird der Bilddetektor 7 im Auslesemodus betrieben, der es gestattet, auftreffende Röntgenstrahlen zu erkennen. Sobald der Röntgenstrahler über die Bedienvorrichtung 6 eingeschaltet wird, wird am Bilddetektor eine der auftreffenden Strahlung entsprechende Spannung erzeugt. Diese wird nach einer analogen Aufbereitung, hier durch den Baustein 15 angedeutet, sowohl einem A/D-Wandler 16 als auch einem Schwellenwertschalter 17 zugeführt. Wird die im Schwellenwertschalter 17 definierte Schwelle überschritten, geht der Taktgenerator 10 in den Aufnahmemodus über. Die dabei durch Integration der Ladungen erzeugten Bilder können im Bildspeicher 18 abgespeichert und über die Bilddarstellungseinheit 14 (Fig. 1) wiedergegeben werden. Entsprechend wird das Ende der Aufnahme erkannt und sodann wieder in den Auslesemodus zurückgeschaltet. Das Umschalten erfolgt in relativ kurzer Zeit; je nach Geschwindigkeit des horizontalen Taktes beträgt die Zeitdauer, bis der Maximalwert der Ausgangsspannung erreicht wird, zwischen etwa 150 und 700 µs bei einer angenommenen Taktfrequenz von 4 MHz bis 1 MHz.

Damit die Detektion der Strahlung auch unter ungünstigen Umständen, z.B. auch bei teilweiser Abdeckung des Registers durch Plomben, sicher erfolgt, ist die Schwelle des Schwellenwertschalters 17 so definiert, daß eine Auslösung weit unter dem Maximalwert liegt. Dadurch kann die Ansprechzeit im Regelfall noch kürzer ausfallen als oben erwähnt.

## Patentansprüche

1. Zahnärztliche Röntgendiagnostikeinrichtung, enthaltend einen Röntgenstrahler (1), einen diametral dazu und in Strahlungsrichtung nach dem zu durchstrahlenden Objekt (3) angeordneten CCD-Bilddetektor (7), eine Steuerelektronik (9) mit einem Steuerteil (10) zur Ansteuerung des Bilddetektors (7) und eine Bildverarbeitungseinheit (11), welche die vom Bilddetektor (7) gewonnenen Signale aufbereitet und einer Bild-darstellungseinheit (14) zuführt, und eine Bedienvorrichtung (6), mit der der Röntgenstrahler (1) ein- und ausschaltbar ist, **dadurch gekennzeichnet**, daß bei abgeschaltetem Röntgenstrahler (1) der CCD-Bilddetektor (7) mittels eines Taktgenerators (10) kontinuierlich ausgelesen wird, wobei die ankommenden Bilddaten ständig einer Schwellenwertüberprüfung unterzogen werden, indem bei abgeschaltetem Röntgenstrahler (1) der Bilddetektor im Auslesemodus betrieben wird und daß bei Vorhandensein von Strahlung und Überschreiten eines definierten Schwellenwertes der Taktgenerator (10) und der Bilddetektor (7) in den Aufnahmemodus geschaltet werden.

## Claims

1. Dental X-ray diagnostic device, containing an X-ray source (1), a CCD image detector (7) arranged diametrically thereto and in the radiation direction towards the object (3) to be penetrated by radiation, control electronics (9) with a control part (10) to activate the image detector (7) and an image processing unit (11), which prepares the signals obtained by the image detector (7) and supplies them to an image display unit (14), and an operating device (6), with which the X-ray source (1) can be switched on and off, characterized in that when the X-ray source (1) is switched off, the CCD image detector (7) is read out continuously by means of a clock generator (10), whereby the arriving image data are constantly subjected to a threshold-value examination, in that when the X-ray source (1) is switched off, the image detector is operated in the readout mode, and in that with the presence of radiation and upon the exceeding of a defined threshold value the clock generator (10) and the image detector (7) are switched into the location mode.

## Revendications

1. Dispositif de diagnostic dentaire par rayons X, comportant une source (1) de rayons X, un capteur (7) d'image CCD disposé diamétralement par rapport à la source et, suivant la direction de rayonnement, après l'objet (3) à irradier, un dispositif (9) électronique de commande comportant une partie (10) de commande destinée à commander le capteur (7) d'image, et une unité (11) de traitement d'image, qui met en forme les signaux extraits du capteur (7) d'image et les envoie à une unité (14) de visualisation d'image, et un dispositif (6) de commande, par lequel la source (1) de rayons X peut être branchée ou débranchée, caractérisé en ce que, lorsque la source (1) de rayons X est débranchée, le capteur (7) d'image CCD peut être lu de manière continue au moyen d'un générateur (10) d'impulsions, les données d'image arrivant étant soumises de manière permanente à un contrôle de valeur de seuil, tandis que, lorsque la source (1) de rayons X est débranchée, le capteur d'image fonctionne en mode de lecture, et en ce que, en présence d'un rayonnement et d'un dépassement d'une valeur de seuil définie, le générateur (10) d'impulsions et le capteur (7) d'image sont branchés en mode de réception.
